(19) Europäisches Patentamt
European Patent Office
Office européen des brevets

(11) EP 0 813 537 B1

(12) **FASCICULE DE BREVET EUROPEEN**

(45) Date de publication et mention de la délivrance du brevet:
**26.06.2002 Bulletin 2002/26**

(21) Numéro de dépôt: **96905922.9**

(22) Date de dépôt: **06.03.1996**

(51) Int Cl.[7]: **C07F 9/40**, A61K 31/66, A61K 7/00, G01N 33/50, C07F 9/58

(86) Numéro de dépôt international:
**PCT/FR96/00353**

(87) Numéro de publication internationale:
**WO 96/27601 (12.09.1996 Gazette 1996/41)**

(54) **DERIVES PHOSPHORYLES DE NITRONES, LEUR PROCEDE DE PREPARATION ET COMPOSITIONS LES CONTENANT**

PHOSPHORYLIERTE DERIVATE VON NITRONEN, VERFAHREN ZU IHRER HERSTELLUNG UND SIE ENTHALTENDE ZUSAMMENSETZUNGEN

PHOSPHORYLATED NITRONE DERIVATIVES, METHOD FOR PREPARING SAME, AND COMPOSITIONS CONTAINING SAID DERIVATIVES

(84) Etats contractants désignés:
**CH DE DK FR GB IE IT LI NL SE**

(30) Priorité: **06.03.1995 FR 9502598**

(43) Date de publication de la demande:
**29.12.1997 Bulletin 1997/52**

(73) Titulaire: **CENTRE NATIONAL DE LA RECHERCHE SCIENTIFIQUE (CNRS)
75016 Paris (FR)**

(72) Inventeurs:
- **ZEGHDAOUI, Abdelhamid
Commune de Bourouba 16230 Algerie (DZ)**
- **FINET, Jean-Pierre
F-13280 Raphele-les-Arles (FR)**
- **TUCCIO, Béatrice
F-13003 Marseille (FR)**
- **CERRI, Viviane
F-83220 Le Pradet (FR)**
- **TORDO, Paul
F-13004 Marseille (FR)**

(74) Mandataire: **Bernasconi, Jean Raymond et al
c/o Cabinet Lavoix,
2, Place d'Estienne d'Orves
75441 Paris Cedex 09 (FR)**

(56) Documents cités:
**WO-A-91/05552
WO-A-95/03314
WO-A-95/11908**

- **J. CHEM. SOC., PERKIN TRANS. 2 (JCPKBH,03009580);95; (12); PP.2087-9, UNIV. PROVENCE;LAB. STRUCTURE REACTIVITE DES ESPECES PARAMAGNETIQUES; MARSEILLE; 13397; FR. (FR), XP002005647 ZEGHDAOUI A*** ET AL: ".beta.-Phosphorylated.alpha.-phenyl-N-ter t-butylnitrone (PBN) analogs: a new series of spin traps for oxyl radicals"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 813 537 B1

Printed by Jouve, 75001 PARIS (FR)

## Description

L'invention a pour objet des dérivés phosphorylés de nitrones utilisables comme pièges à radicaux libres.

De tels composés sont, en raison de leurs propriétés physico-chimiques, très recherchés dans les domaines de la cosmétologie et de la médecine.

Il a été démontré notamment que sous l'action de nombreux processus intracellulaires, l'oxygène véhiculé dans l'organisme par la respiration, peut être réduit en diverses espèces oxygénées réactives, des prooxydants du type $O_2^{\cdot -}$ (le radical superoxyde), $HO^{\cdot}$ (le radical hydroxyle) et $HOO^{\cdot}$ (le radical perhydroxyle).

En situation normale, ces espèces sont rapidement éliminées par réaction avec des substances antioxydantes enzymatiques ou non enzymatiques telles que la catalase, la superoxyde dismutase, la glutathione peroxydase, la glutathione, la vitamine E ou l'ascorbate, de telle sorte que la concentration stationnaire en espèces oxygénées réactives dans les milieux biologiques humains est très faible.

Il arrive cependant qu'un déséquilibre s'établisse entre antioxydants et prooxydants conduisant à une augmentation anormale du taux de prooxydants. On parle alors de situation de stress oxydatif.

Une telle situation a des conséquences néfastes puisque les radicaux libres oxygénés ainsi générés, qui sont très instables, réagissent sans sélectivité aucune avec un grand nombre des composés présents dans le milieu cellulaire, à savoir les protéines, les amino-acides, les carbohydrates, les bases de l'ADN ainsi que les acides organiques et notamment les lipides qui sont des acides gras polyinsaturés. Les dégats résultants entraînent par exemple l'inactivation de nombreuses enzymes ainsi que des modifications au niveau de la perméabilité et de la fluidité des membranes cellulaires.

On a pu mettre en évidence que diverses pathologies cardio-vasculaires telles que l'ischémie coronarienne, l'artériosclérose et l'infarctus impliquent un stress oxydatif, c'est-à-dire une surproduction de radicaux du type $O_2^{\cdot -}$, $HOO^{\cdot}$ et $HO^{\cdot}$. Il en va de même dans le cas de nombreux processus inflammatoires, infectieux ou de vieillissement cellulaire.

Le cerveau plus que tout autre organe est sensible à cet état de stress oxydatif puisque les perturbations dues à la multiplication incontrôlée des radicaux libres oxygénés résultent en une dégénérescence neuronale irréversible.

Or, le cerveau est particulièrement vulnérable dans la mesure où il contient une forte proportion d'acides gras péroxydables ainsi que de nombreuses zones très riches en fer (sous la forme de ferritine), lequel, agissant en tant que catalyseur dans la production des espèces toxiques oxygénées, contribue à aggraver cet état de stress oxydatif. De même, la rareté des substances anti-oxydantes au niveau cérébral n'est pas sans faciliter l'instauration de l'état de stress oxydatif.

On comprend dès lors que la recherche de pièges à radicaux libres efficaces se soit développée. De fait, un moyen simple d'enrayer la multiplication de ces radicaux toxiques est de les mettre en présence avec une substance suffisamment réactive pour entrer en compétition avec les autres constituants cellulaires.

A ce jour, une nitrone déterminée, l'$\alpha$-phényl-tert-butyl-nitrone (PBN), s'est révélée particulièrement efficace comme substance exogène antioxydante apte à renverser les effets néfastes des radicaux libres oxygénés, que ceux-ci aient été générés lors d'une séquence ischémie-reperfusion cérébrale (J.M. Carney et al; J. of Mol. Neurosc. 1991, 3, 45-57) ou suite au vieillissement cérébral (J.M. Carney et al, Proc. Natl. Acad. Sci. USA, 88, 1991, 3633-3636). Toutefois, la faible stabilité des adduits résultants, obtenus par action de la PBN sur les différents radicaux libres, a incité les présents inventeurs à mettre au point de nouvelles molécules également utilisables comme pièges à radicaux libres mais dotées de meilleures propriétés physicochimiques.

A l'issue d'une recherche approfondie, ils ont découvert de nouvelles nitrones phosphorylées présentant une grande stabilité, une bonne solubilité dans les milieux biologiques, une bonne lipophilicité de façon à pouvoir pénétrer la paroi membranaire et conduisant à la formation d'adduits de piégeage comparativement plus stables.

L'invention a donc pour objet des nitrones phosphorylées de formule générale I :

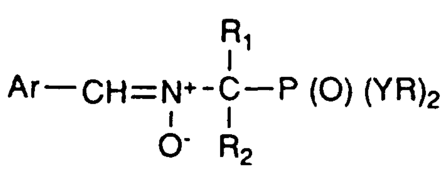

(I)

dans laquelle

$R_1$ et $R_2$ représentent indépendamment un ($C_1$-$C_{18}$) alkyle ou un groupe phényle éventuellement substitué par

($C_1$-$C_{18}$) alkyle, ($C_1$-$C_{18}$) alcoxy ou un atome d'halogène;
Y représente O ou $CH_2$;
R représente un atome d'hydrogène, un groupe ($C_1$-$C_{18}$) alkyle ou un groupe ($C_6$-$C_{18}$)aryle et, lorsque Y représente O, R peut représenter également un métal alcalin;
Ar représente un noyau aromatique choisi parmi un noyau phényle, naphtyle, 2-pyridyle, 3-pyridyle, 4-pyridyle ou un noyau benzo-pyridyle de formule (i)

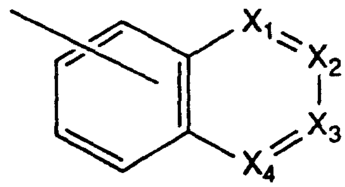

(i)

dans laquelle l'un de $X_1$, $X_2$, $X_3$ et $X_4$ représente un atome d'azote, les autres représentant un atome de carbone, l'atome d'azote endocyclique du noyau 2-pyridyle, 3-pyridyle, 4-pyridyle ou benzo-pyridyle (i) étant éventuellement N-oxydé ou substitué par un groupe alkyle en $C_1$-$C_{18}$ ou un groupe ($C_6$-$C_{18}$) aryle, et, ledit noyau aromatique étant éventuellement C-substitué par un ou plusieurs atomes d'halogène ou un ou plusieurs groupes choisis parmi ($C_1$-$C_{18}$) alkyle; cyano; hydroxyle; ($C_1$-$C_{18}$) alcoxy; ($C_6$-$C_{18}$) aryloxy; carboxy; ($C_1$-$C_{18}$) alcoxycarbonyle; nitro; trifluorométhyle; -$SO_3$M où M est un métal alcalin ou un atome d'hydrogène; amino éventuellement alkylé par un ou deux groupes ($C_1$-$C_{18}$) alkyle;
et $-N^+R_3R_4R_5$ dans lequel $R_3$, $R_4$ et $R_5$ sont choisis indépendamment l'un de l'autre parmi ($C_1$-$C_{18}$) alkyle,

étant entendu que lorsque le groupe Ar comprend un atome d'azote quaternaire, il comprend en outre le contre ion négatif physiologiquement acceptable nécessaire à la neutralité électrique;
ainsi que leurs sels physiologiquement acceptables obtenus par action d'une base ou d'un acide minéral ou organique.

Selon l'invention, on entend par groupe alkyle, à la fois les groupes alkyles linéaires et ceux ramifiés.

De même, le terme alcoxy regroupe les radicaux alcoxy dont la partie alkyle est linéaire mais aussi ceux dont la partie alkyle est ramifiée.

Comme exemple de groupe alkyle en $C_1$-$C_{18}$, on peut citer les groupes méthyle, éthyle, propyle, isopropyle, n-butyle, sec-butyle, tert-butyle, n-pentyle, néopentyle, hexyle, heptyle, décyle, dodécyle, tridécyle et octadécyle.

On entend notamment par groupe alcoxy en $C_1$-$C_{18}$ les groupes méthoxy, éthoxy, n-propoxy, isopropoxy, n-butoxy, sec-butoxy, tert-butoxy, n-pentoxy et néopentoxy, ainsi que les chaînes alcoxy dérivées d'alcool gras.

Quelques exemples de groupes aryle en $C_6$-$C_{18}$ sont les groupes phényle, naphtyle, anthracényle, le dibenzo-1,2,3,4-naphtyle et le pyrényle.

On entend par aryloxy, un groupe aryle, tel que défini ci-dessus, relié à un atome d'oxygène.

Le terme halogène désigne de façon générale le fluor, le chlore, le brome ou l'iode. Lorsque ce substituant halogène est lié au noyau aromatique que représente Ar, on préfère que ce soit un atome de fluor.

Selon l'invention l'expression métal alcalin désigne l'un quelconque des éléments choisis parmi Li, Na, K, Rb et Cs, le sodium et le potassium étant néanmoins préférés.

Lorsque Ar représente le radical benzo-pyridyle de formule (i), ce radical peut être lié au carbone $sp_2$ du composé de formule I par l'un quelconque de ces sommets carbonés, que ce sommet appartienne à l'homocycle ou à l'hétérocycle.

En outre, lorsque Ar comprend un azote quaternaire, la présence d'un contre-ion négatif est nécessaire pour assurer la neutralité électrique.

C'est notamment le cas lorsque Ar représente un noyau pyridyle ou un noyau benzo-pyridyle de formule (i) dans lequel l'atome d'azote endocyclique est alkylé.

C'est également le cas lorsque le noyau aromatique que représente Ar est substitué par un groupe $-N^+R_3R_4R_5$.

La nature du contre-ion n'est pas essentielle selon l'invention, du moment que cet anion est physiologiquement acceptable.

Néanmoins, on peut citer comme contre-ions préférés les halogénure, carbonate, sulfonate, sulfate, phosphate, phosphonate et carboxylates tels que oxalate, citrate, succinate, maléate ou lactate.

On notera également que les composés de formule I dans lesquels $R_1$ est différent de $R_2$ présentent au moins un carbone asymétrique. La présente invention vise dans ce cas non seulement le racémique mais également l'isomère lévogyre et l'isomère dextrogyre de façon spécifique, ainsi que les mélanges de l'isomère lévogyre et de l'isomère dextrogyre en toute proportion.

Les sels des composés de formule I consitutent un autre objet de l'invention.

Lorsque le composé de formule I comprend une fonction acide, on pourra obtenir un sel d'addition avec une base. Des exemples de tels sels comprennent des sels formés avec un métal alcalin ou un métal alcalino-terreux, tel que le sodium, potassium, le calcium ou le magnésium et des sels formés avec une base organique, tels qu'un sel formé avec la dicyclohexylamine.

Lorsque le composé de formule I comprend une fonction basique, on pourra obtenir un sel d'addiditon avec un acide. Des exemples de tels sels d'addition avec des acides comprennent les sels formés avec un acide minéral, tel que l'acide chlorhydrique, l'acide sulfurique ou l'acide phosphorique, les sels formés avec un acide organique carboxylique, tel que l'acide oxalique, l'acide maléique, l'acide succinique ou l'acide citrique, et les sels formés avec un acide organique sulfonique, tels que l'acide méthanesulfonique, l'acide benzène-sulfonique ou l'acide p-toluènesulfonique.

Un premier groupe de composés préférés est constitué des composés de formule I pour lesquels Ar représente un noyau aromatique choisi parmi phényle, 2-pyridyle, 3-pyridyle ou 4-pyridyle éventuellement N-oxydé, ledit noyau aromatique étant éventuellement C-substitué par un atome d'halogène de préférence un atome de fluor, ou un groupe choisi parmi ($C_1$-$C_{18}$) alkyle; cyano; ($C_1$-$C_{18}$) alcoxy; carboxy; ($C_1$-$C_{18}$) alcoxycarbonyle; nitro; trifluorométhyle; $-SO_3M$ où M est un métal alcalin et de préférence le sodium; et
$-N^+(R_3)_3$ dans lequel $R_3$ représente ($C_1$-$C_{18}$) alkyle,
étant entendu que lorsque le groupe Ar comprend un atome d'azote quaternaire, il comprend en outre le contre ion négatif physiologiquement acceptable nécessaire à la neutralité électrique;
ainsi que leurs sels physiologiquement acceptables obtenus par action d'une base ou d'un acide, minéral ou organique.

Un second groupe de composés préférés est constitué des composés de formule I pour lesquels Ar représente phényle éventuellement substitué en position ortho, meta ou para par un atome d'halogène ou un groupe choisi parmi ($C_1$-$C_{18}$)alkyle, nitro, amino, hydroxyle, ($C_1$-$C_{18}$)alcoxy et ($C_6$-$C_{18}$)aryloxy, ainsi que leurs sels physiologiquement acceptables obtenus par action d'une base ou d'un acide minéral ou organique.

Un troisième groupe de composés préférés est constitué des composés de formule I pour lesquels Ar représente un noyau aromatique choisi parmi 2-pyridyle, 3-pyridyle et 4-pyridyle, ledit noyau aromatique étant éventuellement substitué sur l'atome d'azote par un substituant choisi parmi ($C_1$-$C_{18}$) alkyle et ($C_6$-$C_{18}$) aryle, ainsi que leurs sels physiologiquement acceptables obtenus par action d'une base ou d'un acide minéral ou organique.

Un quatrième groupe de composés préférés est constitué des composés de formule I pour lesquels Ar représente un noyau aromatique choisi parmi 2-pyridyle, 3-pyridyle et 4-pyridyle, ledit noyau aromatique étant N-oxydé, ainsi que leurs sels physiologiquement acceptables obtenus par action d'une base ou d'un acide minéral ou organique.

Parmi ces composés ceux pour lesquels Y représente O et $R_1$ et $R_2$ représentent indépendamment ($C_1$-$C_{18}$) alkyle, et mieux encore ($C_1$-$C_5$) alkyle, sont plus particulièrement préférés.

Avantageusement, R représente un groupe ($C_1$-$C_{18}$) alkyle ou un métal alcalin et plus particulièrement le sodium, le potassium, le lithium ou le césium.

Les composés suivants sont plus particulièrement préférés :

le 1-(N-benzylidène-N-oxy)amino-1-méthyléthylphosphonate de diéthyle;
le 1-[N-oxy-N-[4-(N'-oxypyridinyl) formylène]amino]-1-méthyléthylphosphonate de diéthyle;
le 1-[[N-(4-nitrophenyl)formylène]-N-oxyamino]-1-méthyléthyl phosphonate de diéthyle;
le 1-[[N-(4-chlorophényl) formylène] -N-oxyamino] -1-méthyl-éthylphosphonate de diéthyle; et
leur sels physiologiquement acceptables obtenus par action d'une base ou d'un acide, minéral ou organique.

Les composés de l'invention peuvent être préparés de façon générale par réaction d'un composé de formule II

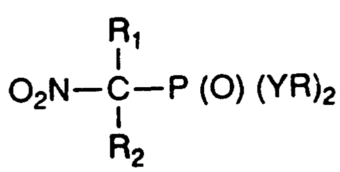

(II)

dans laquelle $R_1$, $R_2$, Y et R ont les significations indiquées ci-dessus, préalablement activé par une suspension de zinc métallique dans une solution aqueuse de chlorure d'ammonium, avec un aldéhyde de formule III

$$Ar - CH = O \qquad (III)$$

dans laquelle Ar est tel que défini ci-dessus. L'étape d'activation du composé de formule II peut être réalisée par mise en oeuvre des étapes suivantes :

(i) dans un premier temps, le composé de formule II ci-dessus est mis à réagir dans un solvant approprié avec du zinc métallique en présence d'une solution aqueuse de chlorure d'ammonium, par exemple à une température comprise entre -10°C et la température ambiante, de préférence entre -5°C et la température ambiante, mieux encore entre 0°C et la température ambiante.
(ii) Puis, le milieu réactionnel est filtré. Le filtrat obtenu, contenant la forme activée du composé de formule II peut être utilisé tel quel dans le procédé décrit ci-dessus consistant à faire réagir le composé de formule II activé sur un composé de formule III.

L'homme du métier n'aura aucun mal à déterminer le solvant approprié à l'étape (i) d'activation. De préférence, le solvant en question sera caractérisé par une forte polarité, sa miscibilité éventuelle à l'eau étant préférable mais non pas essentielle. De préférence, ledit solvant est choisi de façon à solubiliser le dérivé nitré de formule II.
Comme exemple de solvants particulièrement avantageux, on citera les alcools organiques de formule T-OH où T est alkyle en ($C_1$-$C_6$), de préférence alkyle en ($C_1$-$C_4$), éventuellement mono-ou poly-hydroxylé.
A titre d'exemple, on peut mentionner le méthanol et préférablement l'éthanol.
L'utilisation de mélanges de solvants peut s'avérer également très profitable. C'est ainsi que l'on pourra opter pour des mélanges de type alcool/eau et plus particulièrement éthanol/eau.
Le procédé décrit ci-dessus pour l'activation du composé de formule II par le zinc en milieu chlorure d'ammonium aqueux est donné à titre d'exemple en tant que mode de réalisation préféré. L'invention n'entend pas limiter cependant le procédé d'activation à ce mode de réalisation particulier.
La réaction du composé de formule II activé, sur le composé de formule III est mise en oeuvre de préférence à une température comprise entre la température ambiante et 200°C, mieux encore entre la température ambiante et 150°C ou bien entre la température ambiante et 100°C.
Si nécessaire, un solvant peut être ajouté au milieu réactionnel lors de la réaction du composé III sur le composé II activé, et ceci même dans l'hypothèse où l'on utilise comme réactif de départ le filtrat recueilli à l'étape (ii) du procédé d'activation décrit ci-dessus.
Là encore la détermination du solvant approprié est à la portée de l'homme du métier. Un solvant polaire est à nouveau particulièrement indiqué, les alcools organiques mentionnés ci-dessus étant particulièrement avantageux. De façon générale, l'utilisation d'éthanol est recommandée.
On notera que dans le procédé de préparation des composés de formule I, la nature du zinc métallique n'est pas essentielle.
Toutefois, la mise en oeuvre dudit procédé pourra être simplifiée par l'utilisation de zinc en poudre, éventuellement activé selon l'un des procédés bien connu par le chimiste organicien.
La présente invention propose également en variante du procédé précédent, une seconde voie de synthèse des composés de formule I.
Ce second procédé comprend la réaction dans un solvant approprié d'un composé de formule II

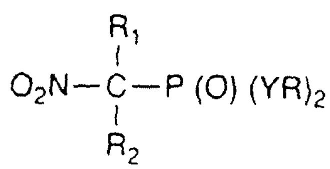
(II)

(dans laquelle $R_1$, $R_2$, Y et R sont tels que définis ci-dessus), sur un composé de formule III

$$Ar - CH = O \qquad (III)$$

(dans laquelle Ar est tel que défini ci-dessus),
en présence de zinc métallique et d'acide acétique.
Cette réaction a lieu de préférence à une température comprise entre -10°C et la température ambiante, mieux encore entre 0°C et la température ambiante.
Pour une efficacité optimale, il est clair que ce procédé sera préférablement sélectionné dans le cas des composés de formule I pour lesquels Ar ne comprend pas de fonctions susceptibles d'être réduites par action de zinc

métallique dans l'acide acétique. Des exemples de telles fonctions sont par exemple la fonction nitro, et $-N^+-O^-$. En effet, suite à la réaction d'un tel composé de formule II sur le composé de formule III, une étape supplémentaire d'oxydation de ladite fonction serait alors nécessaire pour aboutir au composé souhaité de formule I.

Ainsi ce procédé est-il plus particulièrement recommandé pour la préparation des composés de formule I

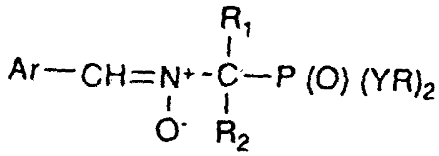

(I)

dans laquelle

$R_1$ et $R_2$ représentent indépendamment un ($C_1$-$C_{18}$) alkyle ou un groupe phényle éventuellement substitué par ($C_1$-$C_{18}$) alkyle, ($C_1$-$C_{18}$) alcoxy ou un atome d'halogène;
Y représente O ou $CH_2$;
R représente un atome d'hydrogène, un groupe ($C_1$-$C_{18}$) alkyle ou un groupe ($C_6$-$C_{18}$) aryle et, lorsque Y représente O, R peut représenter également un métal alcalin;
Ar représente un noyau aromatique choisi parmi un noyau phényle, naphtyle, 2-pyridyle, 3-pyridyle, 4-pyridyle ou un noyau benzo-pyridyle de formule (i)

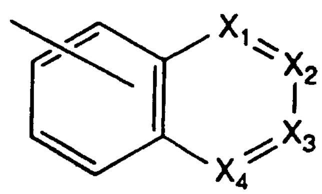

(i)

dans laquelle l'un de $X_1$, $X_2$, $X_3$ et $X_4$ représente un atome d'azote, les autres représentant un atome de carbone, ledit noyau aromatique étant éventuellement C-substitué par un ou plusieurs atomes d'halogène ou un ou plusieurs groupes choisis parmi ($C_1$-$C_{18}$) alkyle; hydroxyle; ($C_1$-$C_{18}$)alkoxy; trifluorométhyle; ($C_6$-$C_{18}$)aryle; carboxy; ($C_1$-$C_{18}$)alcoxycarbonyle; $-SO_3M$ où M est un métal alcalin ou un atome d'hydrogène; amino éventuellement alkylé par un ou deux groupes ($C_1$-$C_{18}$)alkyle; et $-N^+R_3R_4R_5$ dans lequel $R_3$, $R_4$ et $R_5$ sont choisis indépendamment l'un de l'autre parmi ($C_1$-$C_{18}$) alkyle,

étant entendu que lorsque le groupe Ar comprend un atome d'azote quaternaire, il comprend en outre le contre ion négatif physiologiquement acceptable nécessaire à la neutralité électrique;
ainsi que leurs sels physiologiquement acceptables obtenus par action d'une base ou d'un acide minéral ou organique.

Les composés de formule générale II peuvent être préparés en utilisant le procédé décrit par K.A. Petrov et al. dans Zh. Obshch. Khim. 1976, 46, 1226. D'autres méthodes bien connues de l'homme du métier permettent la préparation de ces dérivés nitro-phophorylés.

Quant aux composés de formule III, ils sont pour la plupart disponibles dans le commerce, et dans tous les cas facilement préparés à partir de méthodes connues.

Les composés de l'invention sont des pièges à radicaux libres efficaces qui peuvent être utilisés dans différents domaines :

- en cosmétologie, comme capteurs des espèces prooxydantes responsables du vieillissement cellulaire;
- en thérapeutique en vue de rétablir l'équilibre entre espèces prooxydantes et antioxydantes qui peut avoir été perturbé par exemple suite à un vieillissement cérébral ou lors d'une séquence ischémie-reperfusion cérébrale; les composés de l'invention sont également utilisables pour le traitement des pathologies impliquant une situation de stress oxydatif tels que des pathologies cardio-vasculaires, par exemple l'ischémie coronarienne, l'artériosclérose et l'infarctus; ou bien encore
- dans le domaine du diagnostic, où ils sont utiles dans l'évaluation du stress oxydatif.

De fait, la détection directe des radicaux libres $O_2^{\cdot -}$, $HOO^{\cdot}$ et $HO^{\cdot}$ générés in vivo n'est pas possible par Résonance Paramagnétique Electronique (RPE), malgré leur magnétisme de spin non nul, et ceci en raison de leur instabilité : ces radicaux présentent en effet une durée de demi-vie de l'ordre de $10^{-5}$ à $10^{-3}$ secondes.

Pour cette raison, la technique du spin-trapping est largement utilisée pour la détection de ces radicaux.

Son principe est le suivant : le milieu biologique à tester est placé en présence d'un piège à radicaux libres P. Si des radicaux libres tels que $O_2^{\cdot -}$, $HO^{\cdot}$ ou $HOO^{\cdot}$ sont présents dans le milieu, ils se combinent au piège P pour former un adduit $(P\text{--}O_2)^{\cdot -}$, $(P\text{--}OH)^{\cdot}$ ou $(P\text{--}OOH)^{\cdot}$. Cet adduit est paramagnétique de manière persistante et peut donc être détecté par Résonance Paramagnétique Electronique (RPE).

Or, vis-à-vis des radicaux libres, les composés selon l'invention se sont révélés des pièges efficaces permettant la détection par RPE des radicaux libres prooxydants dans les milieux biologiques, du fait notamment de l'excellente stabilité de l'adduit de piégeage formé, et de l'excellente cinétique de piégeage caractérisant les composés de l'invention.

Comparés à l'α-phényl-tert-butyl nitrone (PBN) de l'état de la technique, les composés de l'invention présentent une meilleure sélectivité vis-à-vis des substances radicalaires oxygénés. Plus particulièrement, ils permettent la détection du radical $O_2^{\cdot -}$, détection qui n'est pas réalisable à partir de PBN.

L'utilisation des composés de l'invention est d'autant plus avantageuse qu'ils sont solubles dans les milieux biologiques tout en étant capables de pénétrer l'espace intra-cellulaire. On pense en effet que les processus radicalaires intra-cellulaires sont prépondérants *in vivo.* On notera qu'en modifiant la nature du groupement Ar, il est possible de moduler la lipophilie de la nitrone synthétisée.

L'invention a également pour objet un composition cosmétique ou pharmaceutique comprenant à titre d'ingrédient actif au moins un composé de l'invention en association avec un véhicule approprié.

Lorsque les compositions de l'invention sont sous forme solide, les composés de l'invention sont associés à différents excipients, arômes, colorants, parfums, lubrifiants, correctifs de goût, filtres UV organiques ou inorganiques ou plus généralement de charges telles que lactose, amidon, saccharose, glucose, sorbitol, cellulose cristalline et dioxyde de silicium.

Comme exemple de liants, on peut citer les poly(alcool vinylique), poly(éther vinylique), éthyl-cellulose, méthylcellulose, acacia, gomme adragante, gélatine, Shellac, hydroxypropyl- celullose, dextrine, pectine, éthanol, propanol et eau.

Le colorant peut être l'un quelconque de ceux appropriés à une utilisation cosmétique ou thérapeutique.

Des exemples de correctifs de goûts englobent le cacao, la menthe, le bornéol et la cannelle.

Comme exemples d'excipients, on citera les huiles végétales ou minérales, les cires végétales ou minérales, les silicones, les alcools et acides gras, les agents tensio-actifs (tels que les esters de sorbitol et leurs dérivés polyoxyéthylénés, les huiles de ricin (hydrogénées ou non) polyoxyéthylénées, les copolymères séquencés oxyde d'éthylène/oxyde de propylène, les alcools gras et les stérols polyoxyéthylénés, le lauryl-sulfate de sodium, le dioctylsulfosuccinate de sodium, les lécithines d'oeuf ou de soja, les huiles de silicones polyoxyéthylénées), les dérivés de protéines, les gélifiants inorganiques ou organiques, la lanoline et ses dérivés, et plus généralement des extraits végétaux.

Pour l'obtention de comprimés, granulés, poudres, capsules, gels, crèmes ou pommades, on procède de façon connue, à la mise en forme du mélange ainsi constitué.

Lorsque les compositions de l'invention sont sous forme de liquide ou de suspension, la nature des véhicules associés diffère. On pourra par exemple utiliser l'eau, l'alcool éthylique, le propylène glycol ou l'alcool isostéarylique. On pourra également ajouter des conservateurs tels que des esters de l'acide 4-hydroxybenzoique.

La quantité d'ingrédient actif devant être ajouté à la composition varie selon que ladite composition est destinée à un usage cosmétologique ou thérapeutique.

Un autre objet de l'invention est une composition de diagnostic utilisable dans l'évaluation du stress oxydatif, comprenant au moins un composé selon l'invention.

Les exemples suivants illustrent l'invention à titre non limitatif. Dans les données de résonance magnétique nucléaire (R.M.N.), les déplacements chimiques δ sont exprimés en p.p.m. par rapport au TMS.

Exemple 1 :

1-(N-benzylidène-N-oxy)amino-1-méthyléthylphosphonate de diéthyle

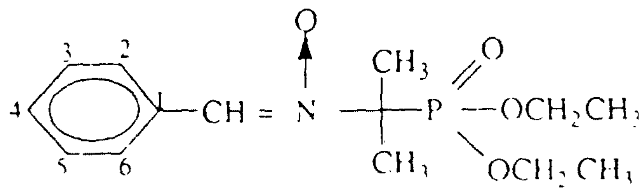

A un mélange vigoureusement agité de benzaldéhyde (10 mmoles), de 1-méthyl-1-nitroéthylphosphonate de diéthyle (préparé selon la procédé décrit dans K.A. Petrov; V.A. Chanzov; L.V. Pastukhova and N.N. Bogdanov, *Zh. Obshch. Khim.,* 1976, 46, 1226), (20 mmoles, 4,5 g) et de zinc en poudre (30 mmoles, 1,96 g) dans 75 ml d'éthanol à 95%, on ajoute goutte à goutte l'acide acétique glacial (60 mmoles, 3,79 g). La température est maintenue à 0°C pendant l'addition. On laisse ensuite revenir à la température ambiante, et on agite pendant 2 à 4 h. Le mélange réactionnel est ensuite laissé une nuit au réfrigérateur. Après filtration et évaporation de l'éthanol sous pression réduite, on obtient un résidu qui est repris dans l'éther (50 ml) et extrait avec une solution aqueuse 1N de $NaHCO_3$, puis avec 50 ml d'eau. La phase aqueuse est réextraite, et les phases organiques réunies sont séchées sur sulfate de sodium anhydre. Après concentration, le résidu est repris dans un minimum de dichlorométhane et chromotographié trois fois sur plaque préparative de silice 60 (éluant : $CH_2Cl_2$-THF-pentane, 1:1.2 en volume).
Rendement : 20%

Le 1-(N-benzylidène-N-oxy) amino-1-méthyléthylphosphonate de diéthyle attendu, isolé sous forme d'huile, est caractérisé par les données spectrales suivantes :

IR $\nu_{max}$ (film)/$cm^{-1}$: 1577 (C=N), 1245 (P=O), 1193 (N-O) et 1163 (P-O-C);
RMN $^1H$ (400 MHz) ($CDCl_3$) : δ 1,22 (6H, dt, $J_{HH}$ 7,6 et $J_{HP}$ 0,54, $OCH_2$-$C\underline{H_3}$), 1,72 (6H, d, $J_{HP}$ 14,89, $C\underline{Me_2}$), 4.09 (4H, dq, $J_{HH}$ 7,6 et $J_{HP}$ 7,1, 0-$\underline{CH_2}$-$CH_3$), 7,28 (2H, m, $H_{3,5}$), 7,29 (1H, m, $H_4$), 7,64 (1H, d, $J_{HP}$ 2,7, $\underline{CH}$=N), 8,17 (2H, dd,*J* 7,6 et 3,4, $H_{2,6}$;
RMN-$^{13}C$ (100,6MHz) ($CDCl_3$) : δ 16,225 (d, $J_{CP}$ 5,7, 0-$CH_2$-$\underline{C}H_3$), 23,093 (s, $CMe_2$), 63,188 (d, $J_{CP}$ 7, O-$\underline{CH_2}$-$CH_3$), 72,704 (d, $J_{CP}$ 153,7, $CMe_2$), 128,210 (s, $C_4$), 128,800 (s, $C_{3,5}$), 130,240 (s, $C_{2,6}$), 130,450 (s, $C_1$), 133,005 (d, $J_{CP}$ 5, $\underline{C}$H = N);
RMN-$^{31}P$ (40,5 MHz) ($CDCl_3$) : δ 22,222

| Analyse : $C_{14}H_{22}NO_4P$, $H_2O$ | | | |
|---|---|---|---|
| calculé | C:52,99; | H: 7,62; | N: 4,41% |
| trouvé | C:52,84; | H: 7,63; | N: 4,22% |

Exemple 2

1-[oxy-N-[4-(N'-oxypyridinyl) formylène] amino]-1-méthyléthyl phosphonate de diéthyle

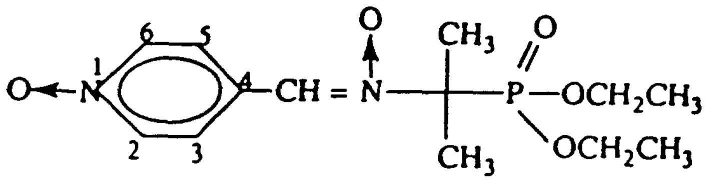

A un mélange vigoureusement agité de 2,25 g (10 mmoles) de 1-méthyl-1-nitroéthylphosphonate de diétyle, 10 ml d'éthanol, 30 ml d'eau, et 10 ml d'une solution aqueuse 2N de chlorure d'ammonium refroidi à 0-10°C, on ajoute 2,5 g de zinc en poudre par petites quantités pendant 2 h. On laisse ensuite revenir à température ambiante et on agite pendant 4 h. Après filtration, on ajoute une solution de 1 g (~6 mmoles) de 1-[4-(N-oxy-pyridyl)]formaldéhyde dans l'éthanol. Le mélange réactionnel est agité à 40-50°C pendant 6 heures. Après concentration de l'éthanol sous pression réduite, le résidu est repris au chloroforme (50 ml) et extrait avec une solution aqueuse 1N de $NaHCO_3$, puis avec 50 ml d'eau. La phase aqueuse est réextraite, et les phases organiques réunies sont séchées sur sulfate de sodium. Après concentration, on purifie le produit obtenu soit par chromatographie soit sur plaque préparative de silice 60 (éluant : $CH_2Cl_2$-TFH-pentane, 1:1:2 en volume) soit par cristallisation (dichlorométhane-pentane, 1:4).
Rendement : 27%.

Le produit attendu présente un point de fusion compris entre 114 et 115°C, et est caractérisé par les données spectrales suivantes :

IR $\nu_{max}$ ($CCl_4$)/$cm^{-1}$: 1569 (C=N), 1241 (P=O), 1164 (N-O) et 1052 (P-O-C);
RMN-$^1$H(400 MHz) ($CDCl_3$) : $\delta$ 1,29 (6H, dt, $J_{HH}$ 7,05 et $J_{HP}$0,5, $OCH_2$-$C\underline{H}_3$), 1,77 (6H, d, $J_{HP}$ 14, 69, $C\underline{Me}_2$), 4,15 (4H, dq, $J_{HH}$ 7,1 et $J_{HP}$ 8, O-$\underline{CH}_2$-$CH_3$), 7,74 (1H, d, $J_{HP}$ 2,7,C$\underline{H}$=N), 8,12 (4H, s, Ar-H);
RMN-$^{13}$C (100,6MHz) ($CDCl_3$) : $\delta$ 16,452 (d, $J_{CP}$ 5,7, O-$CH_2$-$\underline{C}H_3$), 23,147 (s, $C\underline{Me}_2$), 63,653 (d, $J_{CP}$ 7,O-$\underline{CH}_2$-$CH_3$), 73,779 (d, $J_{CP}$ 152,2, $\underline{C}Me_2$), 124,690 (s, $C_{3,5}$), 127,720 (s, $C_4$), 129,670 (d, $J_{CP}$4, $\underline{C}$H = N), 139,010 (s, $C_{2,6}$) ;
ARMN-$^{31}$P (40,5 MHz) ($CDCl_3$):$\delta$ 21,474

| Analyse : $C_{13}H_{21}N_2O_5P$, $H_2O$ | | | |
|---|---|---|---|
| calculé | C:46,71; | H: 6,93; | N: 8,38% |
| trouvé | C,46,83; | H, 6,55; | N, 8,49% |

Exemple 3

1-[[N-(4-nitrophényl) formylène]-N-oxyamino]-1-méthyléthyl phosphonate de diéthyle

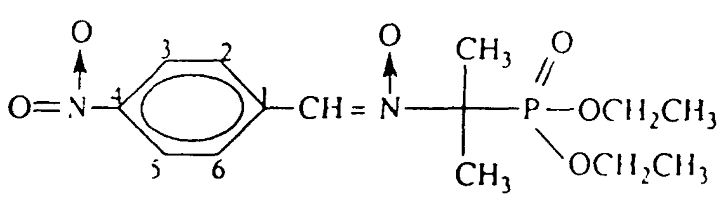

En utilisant le protocole expérimental de l'exemple 2 et en partant de 1-méthyl-1-nitroéthylphosphonate de

diéthyle et de 4-nitrobenzaldéhyde, on isole le produit attendu sous forme d'un solide présentant un point de fusion compris entre 135 et 136°C.

Les données spectrales caractérisant ce composé sont les suivantes :

IR $\nu_{max}$ (film)/$cm^{-1}$: 1545 (C=N), 1484 ($NO_2$), 1247 (P=O), 1176(N->O) et 1150 (P-O-C);
RMN-$^1$H(400 MHz) ($CDCl_3$) : δ 1,30 (6H, dt, $J_{HH}$ 7 et $J_{HP}$3,3, $OCH_2$-$C\underline{H}_3$), 1,82 (6H, d, $J_{HP}$ 14,71, $C\underline{Me}_2$), 4,15 (4H,dq, $J_{HH}$ 7 et $J_{HP}$ 7,6, 0-$\underline{CH}_2$-CH3), 7,88 (1H, d, $J_{HP}$ 2,7, $\underline{CH}$=N), 8,21 (2H, d,$J_{HH}$9,$H_{3,5}$),8,39 (2H, d, $J_{HH}$9,$H_{2,6}$) ;
RMN-$^{13}$C (100,6MHz) ($CDCl_3$): δ 16,476 (d, $J_{CP}$ 5,3, O-$CH_2$-$\underline{C}H_3$), 23,276 (s, $C\underline{Me}_2$), 63,664 (d, $J_{CP}$ 6,4, O-$\underline{C}H_2$-$CH_3$), 74,059 (d, $J_{CP}$ 152,9, $\underline{C}Me_2$), 123,780 (s, $C_{2,5}$), 129,260 (s, $C_{2,6}$), 131,510 (d, $J_{CP}$4, $\underline{C}$H=N), 136,310 (s, $C_1$), 147,830 (s, $C_4$) ;
RMN-$^{31}$P (40,5 MHz) ($CDCl_3$) : δ 21,5

| Analyse : $C_{14}H_{21}N_2O_5P$, $H_2O$ | | | |
|---|---|---|---|
| calculé | C:48,84; | H: 6,15; | N: 8,14% |
| trouvé | C,48,76; | H, 6,05; | N, 8,29% |

Exemple 4

1-[[N- (4-chlorophényl) formylène]-N-oxyamino]-1-méthyléthylphosphonate de diéthyle

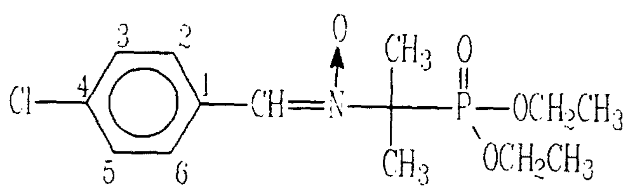

En utilisant le protocole expérimental de l'exemple 2 et en partant de 1-méthyl-1-nitroéthylphosphonate de diéthyle et de 4-chlorobenzaldéhyde, on isole le produit attendu.
P.F. 80-81°C

IR$\nu_{max}$(KBr)$cm^{-1}$: 1570(C=N), 1242 (P=0), 1177(N->O), 1157 (P-O-C) et 1125 (Cl-C)
RMN$^1$H (400 MHz, $CDCl_3$) : δ 1,26 (6H, t, $J_{HH}$ 7,1, O-$CH_2$-$\underline{CH_3}$), 1,76 (6H, d, $J_{HP}$ 14,83, $C\underline{Me}_2$), 4,13 (4H, dq, $J_{HH}$ 7,1 et $J_{HP}$ 7,0-$C\underline{H}_2$-$CH_3$), 7,30(2H, d, AB, J 8,7, $H_{3,5}$), 7,68 (1H, d, $J_{HP}$ 2,7, C$\underline{H}$=N), 8,18 (2H, d, AB, J 8,5, $H_{2,6}$)
RMN$^{13}$C (100,6 MHz, $CDCl_3$) : δ 16,420 (d, $J_{CP}$ 5,9, O-$CH_2$-$\underline{C}H_3$), 23,226 (s, $C\underline{Me}_2$), 63,390 (d, $J_{CP}$ 7,3, 0-$\underline{C}H_2$-$CH_3$), 72,9 (d, $J_{CP}$ 153, $\underline{C}Me_2$), 128,650 (s, $C_{3,5}$), 129,160 (s, $C_1$), 130,130 (s, $C_{2,6}$), 132, 125 (d, $J_{CP}$ 5, $\underline{C}$H=N), 135,750 (s,$C_4$)
RMN$^{31}$P (40,5 MHz, $CDC_3$) : δ 21,98

| $C_{14}H_{21}ClNO_4P$ (M = 333,75) : | | | | |
|---|---|---|---|---|
| calculé | C,50,38; | H,6,34; | N,4,20;; | Cl,10,60% |
| trouvé | C,50,31; | H,6,24; | N,4,14;; | Cl,10,60% |

Exemple 5

En utilisant le protocole expérimental de l'exemple 2 et en partant des réactifs appropriés de formule II et III, on obtient les composés 5.1, 5.2 et 5.3 rapportés dans le tableau suivant.

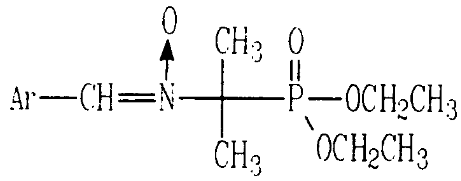

| Composé | 5.1 | 5.2 | 5.3 |
|---|---|---|---|
| Ar | 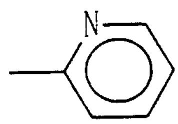 | 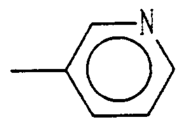 | 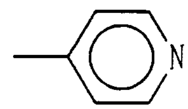 |

Exemple 6

Cet exemple illustre l'influence du groupement Ar par la lipophilie de la nitrone correspondante. Les coefficients de partage $K_p$ des composés des exemples 1, 2 et 4 ont été déterminés dans un mélange biphasique n-octanol/tampon phosphate par analyse spectrométrique UV en utilisant la méthode décrite par Konorev et al. dans Free Rad. Biol. Med. 14 (1993) 127. Les coefficients de partage sont simplement définis comme le rapport de la concentration en nitrone dans la phase lipophile (n-octanol) sur la concentration en nitrone dans la phase aqueuse.

Des solutions 0,25 M dans le 1-octanol des nitrones des exemples 1, 2 et 4 ont été préparées et la concentration en nitrone a été mesurée au maximum d'absorption en utilisant un spectromètre UNICAM UV/visible UV4. Les maximum d'absorption sont respectivement :

$\lambda_{max}$ = 299 nm pour la nitrone de l'exemple 1
$\lambda_{max}$ = 332 nm pour la nitrone de l'exemple 2
$\lambda_{max}$ = 272 nm pour la nitrone de l'exemple 4

Des volumes identiques (2 $cm^3$) de la solution octanolique de nitrone préparée ci-dessus et d'un tampon phosphate 10 mM à pH = 7,4 sont agités vigoureusemnet à 37°C pendant 1 heure puis les deux phases sont séparées par centrifugation (1000 g pendant 20 s). Les concentrations respectives en nitrone dans chaque phase sont alors mesurées.

Les résultats obtenus sont résumés dans le Tableau 2 suivant :

Tableau 2

| Composé | Coefficient de partage |
|---|---|
| ex.1 | 10,1 ± 0,5 |
| ex. 2 | 195 ± 10 |
| ex.4 | 0,18 ± 0,02 |

Il résulte de ces mesures que les nitrones des exemples 1 et 2 sont lipophiles, alors que la nitrone de l'exemple 4 est hydrophile.

Une modulation des propriétés lipophiles des nitrones de l'invention est donc possible par simple modification du groupement Ar.

Exemple 7

Dans cet exemple, les spectres de résonance paramagnétique électronique (RPE) dérivés de la nitrone de

l'exemple 1 (désignée PPN dans le suite) après piégeage des radicaux $HO^{\cdot}$ et $O_2^{\cdot -}$/$HOO^{\cdot}$ sont discutés.

Dans cette expérience les radicaux $HO^{\cdot}$ ont été produits selon la méthode de Fenton dans un tampon phosphate 0,1 M à pH = 7 en présence du système $H_2O_2$-$FeSO_4$. Cette réaction classique de production des radicaux $HO^{\cdot}$ peut-être schématisée comme suit :

$$H_2O_2 + Fe(II) - HO^{\cdot} + HO- + Fe(III).$$

$FeSO_4$ (2 mmol.dm$^{-3}$) et $H_2O_2$ (2 mmol.dm$^{-3}$) sont ajoutés à une solution d'un tampon phosphate 0,1 M contenant 0,1 mol.dm$^{-3}$ de la nitrone de l'exemple 1. Le spectre RPE de l'adduit de piégeage est enregistré environ 40 s après addition du sulfate ferreux.

Pour la production des radicaux $HOO^{\cdot}$, on utilise le système hypoxanthine/xanthine oxydase dans un tampon phosphate 0,1 M maintenu à température ambiante. L'oxygène conduisant à la formation des radicaux $HOO^{\cdot}$ est celui initialement dissous dans le tampon phosphate 0,1 M.

Le système hypoxanthine/xanthine oxydase contient 0,1 M d'un tampon phosphate, 0,4 M d'hypoxanthine, 1 mM d'acide diéthylène-triamine pentaacétique (DTPA), 0,4 unités.cm$^{-3}$ de xanthine oxydase (commercialisée par la société Boehringer Mannheim Biochemica Co.) et 0,1 M de la nitrone testée. Les spectres RPE des adduits de piégeage sont enregistrés environ 40 s après l'addition de xanthine oxydase.

Le spectre RPE du mélange révèle la formation des adduits de piégeage PPN-OH et PPN-$O_2$H dont les formules sont respectivement

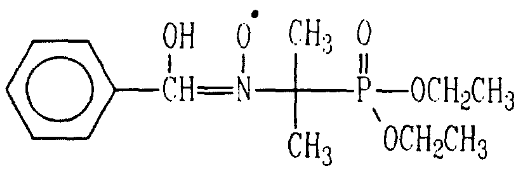

PPN-OH

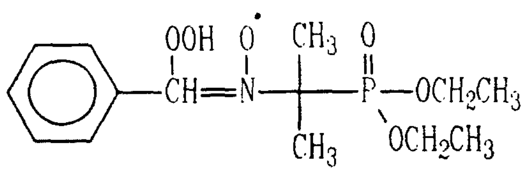

PPN-OOH

La stabilité de ces adduits permet la mesure des constantes de couplage respectives de l'électron célibataire avec les noyaux d'azote ($A_N$), d'hydrogène en β de l'atome d'azote ($A_H$) et de phosphore ($A_P$). Ces valeurs ont été rassemblées dans le tableau 3 suivant :

Tableau 3

| | PPN-OH | PPN-OOH |
|---|---|---|
| $A_N$ | 2, 7 G | 2, 1 G |
| $A_H$ | 14,6 G | 13,5 G |
| $A_p$ | 43,2 G | 41,3 G |

Note : G facteur de Landé

La même expérience a été réalisée dans des conditions identiques à partir de l'α-phényl-tert-butylnitrone (PBN) décrite dans l'état de la technique. Aucun signal n'a pu être détecté par RPE.

L'avantage des nitrones de l'invention sur les composés de l'art antérieur est clair : non seulement les adduits de piégeage sont plus stables mais encore une analyse RPE permet de différencier d'adduit PPN-OH de l'adduit

PPN-OOH.

Exemple 8

Cet exemple rapporte les résultats d'une étude cinétique de la décomposition des adduits résultant du piégeage du radical $HOO^{\cdot}$ à partir des nitrones des exemples 1,2 et 4 de l'invention et des pièges à radicaux libres de l'art antérieur, à savoir la PPB (l'α-phényl-tert-butyl-nitrone), la POBN (α-(1-oxydopyridin-1-ium-4-yl)-N-tert-butylnitrone et la DMPO (la 5,5-diméthylpyrroline-N-oxyde).

Décroissance dans un milieu tampon phosphate

Les adduits de piégeage avec le radical superoxyde ont été produits par le système riboflavine-DTPA (acide diéthylènetriaminepentaacétique) :

Ce système contient 1 M de DTPA, 0,25 M de riboflavine et 0,1 M de nitrone dans un tampon phosphate 0,1 M à pH = 5,8.

Ce milieu est transféré dans la cellule d'un spectromètre VARIAN E-9. La cellule est alors irradiée pour la génération in situ du radical superoxyde. En fin d'irradiation la décroissance des adduits (nitrone-OOH) est étudiée par analyse RPE.

Les résultats des mesures sont rapportées dans le tableau suivant dans le cas des nitrones des exemples 1 et 2 de l'invention, de la POBN et du DMPO. Les cinétiques de disparition des adduits sont dans chaque cas du premier ordre.

Tableau 4

| Nitrone | Constante cinétique k ($10^3.s^{-1}$) | Temps de demi-vie (s) |
|---|---|---|
| exemple 1 | 2,26± 0,03 | 307 |
| exemple 2 | 1,63 ± 0,02 | 425 |
| POBN | 37 ± 2 | 19 |
| DMPO | 10,3 ± 0,3 | 67 |

On a observé que les adduits dérivés des nitrones des exemples 1, 2 de l'invention se dégradent environ 20 fois moins vite que que les adduits POBN-OOH.

De même, les adduits de l'invention se dégradent beaucoup plus lentement que l'adduit DMPO-OOH dans les mêmes conditions.

Décroissance dans un milieu organique

Les adduits de piégeage avec le radical superoxyde ont été produits par le système lumiflavine-DTPA dans la pyridine, respectivement le diméthylformamide. Celui-ci contient 1 M de DTPA, 0,25 M de lumiflavine et 0,1 M de nitrone dans de la pyridine ou du diméthylformamide.

Le protocole opératoire suivi pour l'étude de la décroissance de l'adduit de piégeage est le même que ci-dessus.

Les résultats obtenus sont résumés dans le tableau 5.

Tableau 5

| Nitrone | Solvant | k($10^3.s^{-1}$) | Temps de demi-vie (s) |
|---|---|---|---|
| exemple 1 | DMF | 0,836 ± 0,001 | 829 |
| | pyrridine | 0,608 ± 0,002 | 1140 |
| exemple 2 | DMF | 0,714 ± 0,007 | 971 |
| | pyrridine | 0,549 ± 0,003 | 1263 |
| exemple 4 | DMF | 0,761 ± 0,003 | 911 |
| | pyrridine | 0,573 ± 0,001 | 1210 |

Tableau 5 (suite)

| Nitrone | Solvant | k($10^3.s^{-1}$) | Temps de demi-vie (s) |
|---|---|---|---|
| PBN | DMF | 2,76 ± 0,04 | 251 |
| | pyrridine | 2,62 ± 0,14 | 265 |
| POBN | DMF | 2,29 ± 0,02 | 303 |
| | pyrridine | 2,21 ± 0,19 | 314 |
| DMPO | DMF | 1,53 ± 0,02 | 453 |
| | pyrridine | 1,36 ± 0,02 | 510 |

Il résulte du tableau 5 que les adduits dérivés de nitrones des exemples 1, 2 et 4 se dégradent environ trois fois plus lentement que les adduits POBN-OOH et PBN-OOH.

De même, les adduits de l'invention se dégradent beaucoup plus lentement que l'adduit DMPO-OOH.

A l'inverse des nitrones acycliques disponibles dans l'art antérieur, les nitrones de l'invention sont des pièges efficaces du radical superoxyde non seulement dans les milieux organiques mais aussi dans les milieux aqueux. Les composés de l'invention conduisent notament à des adduits stables donnant naissance à des signaux intenses en RPE.

Les nitrones de l'invention permettent également le piégeage de différents radicaux carbonés tels que $CH_3^{\cdot}$, $HOCH_2^{\cdot}$, $HO(CH_3)CH^{\cdot}$ et $HOOC^{\cdot}$.

## Revendications

1. Composés de formule générale I :

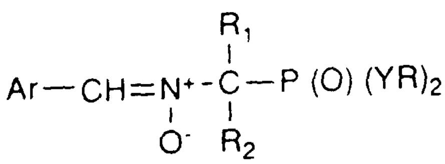

(I)

dans laquelle

$R_1$ et $R_2$ représentent indépendamment un groupe ($C_1$-$C_{18}$)alkyle ou un groupe phényle éventuellement substitué par ($C_1$-$C_{18}$)alkyle, ($C_1$-$C_{18}$)alcoxy ou un atome d'halogène;
Y représente O ou $CH_2$;
R représente un atome d'hydrogène, un groupe ($C_1$-$C_{18}$)alkyle ou un groupe ($C_6$-$C_{18}$)aryle et, lorsque Y représente O, R peut représenter également un métal alcalin;
Ar représente un noyau aromatique choisi parmi un noyau phényle, naphtyle, 2-pyridyle, 3-pyridyle, 4-pyridyle ou un noyau benzo-pyridyle de formule (i)

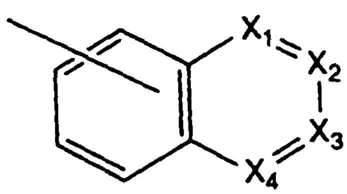

(i)

dans laquelle l'un de $X_1$, $X_2$, $X_3$ et $X_4$ représente un atome d'azote, les autres représentant un atome de carbone, l'atome d'azote endocyclique du noyau 2-pyridyle, 3-pyridyle, 4-pyridyle ou benzo-pyridyle (i) étant éventuellement N-oxydé ou substitué par un groupe alkyle en $C_1$-$C_{18}$ ou un groupe ($C_6$-$C_{18}$) aryle, et, ledit noyau aromatique étant éventuellement C-substitué par un ou plusieurs atomes d'halogène ou un ou plusieurs groupes choisis parmi ($C_1$-$C_{18}$) alkyle; cyano; hydroxyle; ($C_1$-$C_{18}$) alcoxy; ($C_6$-$C_{18}$) aryloxy; carboxy; ($C_1$-$C_{18}$)

alcoxycarbonyle; nitro; trifluorométhyle; $-SO_3M$ où M est un métal alcalin ou un atome d'hydrogène; amino éventuellement alkylé par un ou deux groupes $(C_1-C_{18})$alkyle; et $-N^+R_3R_4R_5$ dans lequel $R_3$, $R_4$ et $R_5$ sont choisis indépendamment l'un de l'autre parmi $(C_1-C_{18})$alkyle,

étant entendu que lorsque le groupe Ar comprend un atome d'azote quaternaire, il comprend en outre le contre ion négatif physiologiquement acceptable nécessaire à la neutralité électrique;
ainsi que leurs sels physiologiquement acceptables obtenus par action d'une base ou d'un acide, minéral ou organique.

**2.** Composés selon la revendication 1 de formule I dans laquelle Ar représente un noyau aromatique choisi parmi phényle, 2-pyridyle, 3-pyridyle ou 4-pyridyle éventuellement N-oxydé, ledit noyau aromatique étant éventuellement C-substitué par un atome d'halogène de préférence un atome de fluor, ou un groupe choisi parmi $(C_1-C_{18})$ alkyle; cyano; $(C_1-C_{18})$ alcoxy; carboxy; $(C_1-C_{18})$ alcoxycarbonyle; nitro; trifluorométhyle; $-SO_3M$ où M est un métal alcalin et de préférence le sodium; et $-N^+(R_3)_3$ dans lequel $R_3$ représente $(C_1-C_{18})$ alkyle,
étant entendu que lorsque le groupe Ar comprend un atome d'azote quaternaire, il comprend en outre le contre ion négatif physiologiquement acceptable nécessaire à la neutralité électrique;
ainsi que leurs sels physiologiquement acceptables obtenus par action d'une base ou d'un acide, minéral ou organique.

**3.** Composés selon l'une quelconque des revendications 1 ou 2, **caractérisé en ce que** Ar représente phényle éventuellement substitué en position ortho, méta ou para par un atome d'halogène ou un groupe choisi parmi $(C_1-C_{18})$ alkyle, nitro, amino, hydroxyle, $(C_1-C_{18})$ alcoxy et $(C_6-C_{18})$ aryloxy,
ainsi que leurs sels physiologiquement acceptables obtenus par action d'une base ou d'un acide minéral ou organique.

**4.** Composés selon l'une quelconque des revendications 1 ou 2, **caractérisé en ce que** Ar représente un noyau aromatique choisi parmi 2-pyridyle, 3-pyridyle et 4-pyridyle, ledit noyau aromatique étant éventuellement substitué sur l'atome d'azote par un substituant choisi parmi $(C_1-C_{18})$alkyle et $(C_6-C_{18})$aryle, ainsi que leurs sels physiologiquement acceptables obtenus par action d'une base ou d'un acide minéral ou organique.

**5.** Composés selon l'une quelconque des revendications 1 ou 2, **caractérisé en ce que** Ar représente un noyau aromatique choisi parmi 2-pyridyle, 3-pyridyle et 4-pyridyle, ledit noyau aromatique étant N-oxydé,
ainsi que leurs sels physiologiquement acceptables obtenus par action d'une base ou d'un acide minéral ou organique.

**6.** Composés selon l'une quelconque des revendications précédentes, de formule I dans laquelle $R_1$ et $R_2$ représentent indépendamment $(C_1-C_{18})$ alkyle, et de préférence $(C_1-C_5)$alkyle et Y représente O,
ainsi que leurs sels physiologiquement acceptables obtenus par action d'une base oud'un acide, minéral ou organique.

**7.** Composés selon l'une quelconque des revendications 1, 2 ou 4, de formule I dans laquelle lorsque Ar comprend un atome d'azote quaternaire, le contre-ion est choisi parmi un halogénure, un carbonate, un sulfonate, un sulfate, un phosphate, un phosphonate et un carboxylate tel qu'un oxalate, un maléate, un citrate, un succinate ou un lactate.

**8.** Composés selon la revendication 1, choisi parmi :

le 1-(N-benzylidène-N-oxy)amino-1-méthyléthylphosphonate de diéthyle;
le 1-[N-oxy-N-[4-(N'-oxypyridinyl)formylène]amino]-1-méthyléthylphosphonate de diéthyle;
le 1-[[N-(4-nitrophényl)formylène]-N-oxyamino]-1-méthyléthyl phosphonate de diéthyle;
le 1-[[N-(4-chlorophényl)formylène]-N-oxyamino]-1-méthyléthylphosphonate de diéthyle; et
leur sels physiologiquement acceptables obtenus par action d'une base ou d'un acide, minéral ou organique.

**9.** Procédé pour la préparation des composés de formule générale I

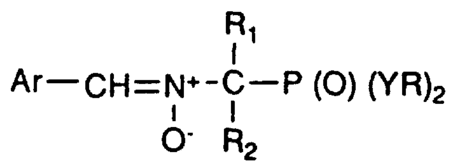 (I)

dans laquelle

$R_1$ et $R_2$ représentent indépendamment un ($C_1$-$C_{18}$) alkyle ou un groupe phényle éventuellement substitué par ($C_1$-$C_{18}$)alkyle, ($C_1$-$C_{18}$) alcoxy ou un atome d'halogène;
Y représente O ou $CH_2$;
R représente un atome d'hydrogène, un groupe ($C_1$-$C_{18}$) alkyle ou un groupe ($C_6$-$C_{18}$) aryle et, lorsque Y représente O, R peut représenter également un métal alcalin
Ar représente un noyau aromatique choisi parmi un noyau phényle, naphtyle, 2-pyridyle, 3-pyridyle, 4-pyridyle ou un noyau benzo-pyridyle de formule (i)

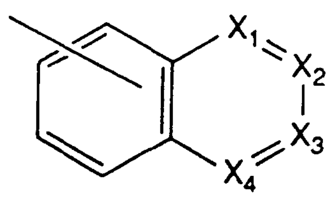 (i)

dans laquelle l'un de $X_1$, $X_2$, $X_3$ et $X_4$ représente un atome d'azote, les autres représentant un atome de carbone, l'atome d'azote endocyclique du noyau 2-pyridyle, 3-pyridyle, 4-pyridyle ou benzo-pyridyle (i) étant éventuellement N-oxydé ou substitué par un groupe alkyle en $C_1$-$C_{18}$ ou un groupe ($C_6$-$C_{18}$) aryle, et, ledit noyau aromatique étant éventuellement C-substitué par un ou plusieurs atomes d'halogène ou un ou plusieurs groupes choisis parmi ($C_1$-$C_{18}$) alkyle; cyano; hydroxyle; ($C_1$-$C_{18}$) alcoxy; ($C_6$-$C_{18}$) aryloxy; carboxy; ($C_1$-$C_{18}$) alcoxycarbonyle; nitro; trifluorométhyle; -$SO_3M$ où M est un métal alcalin ou un atome d'hydrogène; amino éventuellement alkylé par un ou deux groupes ($C_1$-$C_{18}$) alkyle;

et -$N^+R_3R_4R_5$ dans lequel $R_3$, $R_4$ et $R_5$ sont choisis indépendamment l'un de l'autre parmi ($C_1$-$C_{18}$) alkyle,
étant entendu que lorsque le groupe Ar comprend un atome d'azote quaternaire, il comprend en outre le contre ion négatif physiologiquement acceptable nécessaire à la neutralité électrique;
ainsi que leurs sels physiologiquement acceptables obtenus par action d'une base ou d'un acide, minéral ou organique
**caractérisé en ce que** l'on fait réagir dans un solvant approprié un composé de formule II :

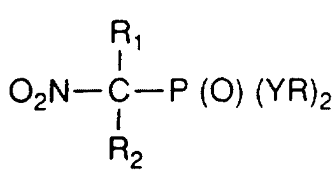 (II)

(dans laquelle $R_1$, $R_2$, Y et R sont tels que définis ci-dessus) préalablement activé par une suspension de zinc métallique dans une solution aqueuse de chlorure d'ammonium, avec un aldéhyde de formule III :

$$Ar\text{-}CH = O \qquad (III)$$

(dans laquelle Ar est tel que défini ci-dessus).

**10.** Procédé selon la revendication 9, **caractérisé en ce que** l'on fait réagir le composé de formule II, préalablement activé, avec un composé de formule III, à une température comprise entre la température ambiante et 200°C, de préférence entre la température ambiante et 150°C.

**11.** Procédé selon l'une quelconque des revendications 9 ou 10, **caractérisé en ce que** l'activation préalable du composé de formule II comprend les étapes consistant à :

(i) faire réagir dans un solvant approprié le composé de formule II avec du zinc métallique en présence d'une solution aqueuse de chlorure d'ammonium, à une température comprise entre -10°C et la température ambiante;
(ii) filtrer le milieu réactionnel pour obtenir une solution homogène du composé de formule II activé, ladite solution étant utilisée telle quelle dans le procédé selon l'une quelconque des revendications 9 ou 10.

**12.** Procédé selon la revendication 11, **caractérisé en ce que** le solvant de l'étape d'activation est un mélange éthanol/eau.

**13.** Procédé pour la préparation de composés de formule générale I

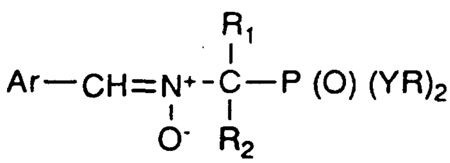

(I)

dans laquelle

$R_1$ et $R_2$ représentent indépendamment un ($C_1$-$C_{18}$) alkyle ou un groupe phényle éventuellement substitué par ($C_1$-$C_{18}$) alkyle, ($C_1$-$C_{18}$) alcoxy ou un atome d'halogène;
Y représente O ou $CH_2$;
R représente un atome d'hydrogène, un groupe ($C_1$-$C_{18}$) alkyle ou un groupe ($C_6$-$C_{18}$) aryle et, lorsque Y représente O, R peut représenter également un métal alcalin,
Ar représente un noyau aromatique choisi parmi un noyau phényle, naphtyle, 2-pyridyle, 3-pyridyle, 4-pyridyle ou un noyau benzo-pyridyle de formule (i)

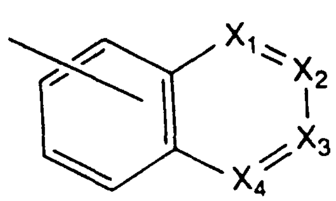

(i)

dans laquelle l'un de $X_1$, $X_2$, $X_3$ et $X_4$ représente un atome d'azote, les autres représentant un atome de carbone, ledit noyau aromatique étant éventuellement C-substitué par un ou plusieurs atomes d'halogène ou un ou plusieurs groupes choisis parmi ($C_1$-$C_{18}$)alkyle; hydroxyle; ($C_1$-$C_{18}$)alcoxy; trifluorométhyle; ($C_6$-$C_{18}$) aryloxy; carboxy; ($C_1$-$C_{18}$)alcoxycar- bonyle; $-SO_3M$ où M est un métal alcalin ou un atome d'hydrogène; amino éventuellement alkylé par un ou deux groupes ($C_1$-$C_{18}$)alkyle; et $-N^+R_3R_4R_5$ dans lequel $R_3$, $R_4$ et $R_5$ sont choisis indépendamment l'un de l'autre parmi ($C_1$-$C_{18}$) alkyle,

étant entendu que lorsque le groupe Ar comprend un atome d'azote quaternaire, il comprend en outre le contre ion négatif physiologiquement acceptable nécessaire à la neutralité électrique;
ainsi que leurs sels physiologiquement acceptables obtenus par action d'une base ou d'un acide minéral ou organique,
**caractérisé en ce qu'**il comprend la réaction dans un solvant approprié d'un composé de formule II

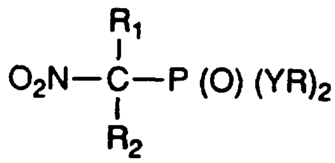

(II)

(dans laquelle $R_1$, $R_2$, Y et R sont tels que définis cu-dessus)
avec un composé de formule III

$$Ar - CH = O \qquad (III)$$

(dans laquelle Ar est tel que défini ci-dessus) en présence de zinc métallique et d'acide acétique.

**14.** Procédé selon la revendication 13, **caractérisé en ce que** la réaction a lieu à une température comprise entre -10°C et la température ambiante.

**15.** Composition cosmétique ou pharmaceutique comprenant à titre d'ingrédient actif au moins un composé selon l'une quelconque des revendications 1 à 8.

**16.** Composition de diagnostic utilisable dans l'évaluation du stress oxydatif, **caractérisée en ce qu'**elle comprend au moins un composé selon l'une quelconque des revendications 1 à 8 .

**Patentansprüche**

**1.** Zusammensetzungen der allgemeinen Formel I:

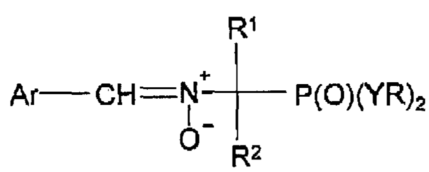

(I)

in der $R_1$ und $R_2$ unabhängig voneinander eine ($C_1$-$C_{18}$) Alkylgruppe oder Phenylgruppe, die eventuell mit ($C_1$-$C_{18}$) Alkyl, ($C_1$-$C_{18}$) Alkoxy oder einem Halogenatom substituiert ist, darstellen;

Y stellt O oder $CH_2$ dar;

R ein Wasserstoffatom, eine ($C_1$-$C_{18}$) Alkylgruppe oder eine ($C_6$-$C_{18}$) Arylgruppe darstellt und, wenn Y O darstellt, kann R auch ein Alkalimetall darstellen;

Ar stellt einen aromatischen Ring dar, ausgewählt aus einem Phenyl-, Naphthyl-, 2-Pyridyl-, 3-Pyridyl-, 4-Pyridyl-Ring oder einem Benzopyridyl-Ring der Formel (i)

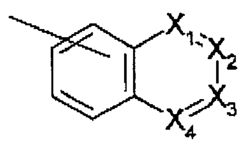

(i)

in der eines von $X_1$, $X_2$, $X_3$ und $X_4$ ein Stickstoffatom darstellt, die anderen ein Kohlenstoffatom darstellen, das endocyclische Stickstoffatom vom 2-Pyridyl-, 3-Pyridyl-, 4-Pyridyl- oder Benzopyridyl-Ring (i) ist möglicherweise ein N-Oxid oder substituiert durch eine Alkylgruppe mit $C_1$-$C_{18}$ oder durch eine ($C_6$-$C_{18}$) Arylgruppe, und, besagter aromatischer Ring ist möglicherweise C-substituiert durch eine oder mehrere Halogenatome oder ein oder mehrere Gruppen ausgewählt aus ($C_1$-$C_{18}$) Alkyl; Cyano; Hydroxyl; ($C_1$-$C_{18}$) Alkoxy; ($C_6$-$C_{18}$) Aryl-oxy; Carboxy; ($C_1$-$C_{18}$) Alkoxycarbonyl; Nitro; Trifluormethyl; -$SO_3M$, wobei M ein Alkalimetall oder ein Wasserstoffatom ist; Amino möglicherweise alkyliert durch ein oder zwei ($C_1$-$C_{18}$) Alkylgruppen; und -$N^+R_3R_4R_5$, in der $R_3$, $R_4$, und $R_5$ unabhängig voneinander ausgewählt werden aus ($C_1$-$C_{18}$) Alkyl,

wobei es als vereinbart gilt, dass wenn die Ar-Gruppe ein quartäres Stickstoffatom umfasst, umfasst es, notwendig für die elektrische Neutralität, außerdem das negative physiologisch akzeptable Gegenion;
genauso wie ihre physiologisch akzeptablen Salze durch Einwirken einer Base oder einer Säure mineralischer oder organischer Natur erhalten werden.

**2.** Zusammensetzungen nach Anspruch 1, der Formel I, in der Ar einen aromatischen Ring darstellt, ausgewählt aus Phenyl, 2-Pyridyl, 3-Pyridyl oder 4-Pyridyl, möglicherweise N-Oxid, besagter aromatischer Ring ist möglicherweise C-substituiert durch ein Halogenatom, bevorzugt ein Fluoratom, oder eine Gruppe ausgewählt aus ($C_1$-$C_{18}$) Alkyl; Cyano; ($C_1$-$C_{18}$) Alkoxy; Carboxy; ($C_1$-$C_{18}$) Alkoxycarbonyl; Nitro; Trifluormethyl; - $SO_3M$, wobei M ein Alkalimetall ist und davon bevorzugt Natrium; und - $N^+(R_3)_3$ in der $R_3$ ($C_1$-$C_{18}$) Alkyl darstellt,
wobei es als vereinbart gilt, dass wenn die Ar-Gruppe ein quartäres Stickstoffatom umfasst, umfasst es, notwendig für die elektrische Neutralität, außerdem das negative physiologisch akzeptable Gegenion;
genauso wie ihre physiologisch akzeptablen Salze durch Einwirken einer Base oder einer Säure mineralischer oder organischer Natur erhalten werden.

**3.** Zusammensetzungen nach irgendeinem der Ansprüche 1 oder 2, **gekennzeichnet dadurch, dass** Ar Phenyl darstellt, möglicherweise substituiert in der *ortho-, meta-* oder *para*-Position durch ein Halogenatom oder eine Gruppe ausgewählt aus ($C_1$-$C_{18}$) Alkyl, Nitro, Amino, Hydroxyl, ($C_1$-$C_{18}$) Alkoxy und ($C_6$-$C_{18}$) Aryloxy,
genauso wie ihre physiologisch akzeptablen Salze durch Einwirken einer Base oder einer Säure mineralischer oder organischer Natur erhalten werden.

**4.** Zusammensetzungen nach irgendeinem der Ansprüche 1 oder 2, **gekennzeichnet dadurch, dass** Ar einen aromatischen Ring darstellt, ausgewählt aus 2-Pyridyl, 3-Pyridyl und 4-Pyridyl, besagter aromatischer Ring ist möglicherweise substituiert über das Stickstoffatom durch einen Substituenten ausgewählt aus ($C_1$-$C_{18}$) Alkyl und ($C_6$-$C_{18}$) Aryl,
genauso wie ihre physiologisch akzeptablen Salze durch Einwirken einer Base oder einer Säure mineralischer oder organischer Natur erhalten werden.

**5.** Zusammensetzungen nach irgendeinem der Ansprüche 1 oder 2, **gekennzeichnet dadurch, dass** Ar einen aromatischen Ring darstellt, ausgewählt aus 2-Pyridyl, 3-Pyridyl und 4-Pyridyl, besagter aromatischer Ring enthält N-Oxid,
genauso wie ihre physiologisch akzeptablen Salze durch Einwirken einer Base oder einer Säure mineralischer oder organischer Natur erhalten werden.

**6.** Zusammensetzungen ausgewählt nach einem der vorhergehenden Ansprüche, der Formel I, in der $R_1$ und $R_2$ unabhängig voneinander ($C_1$-$C_{18}$) Alkyl darstellen, und davon bevorzugt ($C_1$-$C_5$) Alkyl, und Y O darstellt,
genauso wie ihre physiologisch akzeptablen Salze durch Einwirken einer Base oder einer Säure mineralischer oder organischer Natur erhalten werden.

**7.** Zusammensetzungen nach irgendeinem der Ansprüche 1, 2 oder 4, der Formel I in der, wenn Ar ein quartäres Stickstoffatom umfasst, das Gegenion ausgewählt wird aus einem Halogenid, einem Carbonat, einem Sulfonat,

einem Sulfat, einem Phosphat, einem Phosphonat, und einem Carboxylat, sowie einem Oxalat, einem Maleat, einem Citrat, einem Succinat oder einem Lactat.

**8.** Zusammensetzungen nach Anspruch 1, ausgewählt aus:

1-(N-Benzyliden-N-oxy) amino-1-methylethylphosphonsäurediethylester;

1-[N-Oxi-N-[4-(N'-oxypyridinyl) formylen] amino]methylethylphosphonsäure-diethylester;

1-[[N-(4-Nitrophenyl) formylen]N-oxyamino]-1-methylethylphosphonsäure-diethylester;

1-[[N-(4-Chlorophenyl) formylen]-N-oxyamino]-1-methylethylphosphonsäure-diethylester; und

ihre physiologisch akzeptablen Salze, erhalten durch Einwirken einer Base oder einer Säure mineralischer oder organischer Natur.

**9.** Verfahren zur Herstellung von Zusammensetzungen der allgemeinen Formel I,

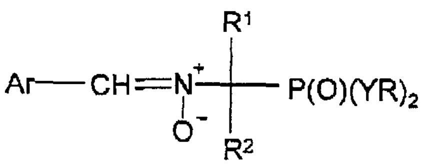

(I)

in der $R_1$ und $R_2$ unabhängig voneinander ein $(C_1-C_{18})$ Alkyl oder eine Phenylgruppe möglicherweise substituiert durch $(C_1-C_{18})$ Alkyl, $(C_1-C_{18})$ Alkoxy oder ein Halogenatom, darstellt;

Y stellt O oder $CH_2$ dar;

R ein Wasserstoffatom, eine $(C_1-C_{18})$ Alkylgruppe oder eine $(C_6-C_{18})$ Arylgruppe darstellt und wenn Y O darstellt, R auch ein Alkalimetall darstellen kann

Ar stellt einen aromatischen Ring dar, ausgewählt aus einem Phenyl-, Naphthyl-, 2-Pyridyl-, 3-Pyridyl-, 4-Pyridylring oder einem Benzopyridylring der Formel (i)

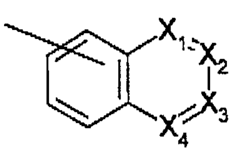

(i)

in der eines von $X_1$, $X_2$, $X_3$ und $X_4$ ein Stickstoffatom darstellt, die anderen ein Kohlenstoffatom darstellen, das endocyclische Stickstoffatom vom 2-Pyridyl-, 3-Pyridyl-, 4-Pyridyl- oder Benzopyridylring (i) ist möglicherweise N-Oxid

oder substituiert durch eine Alkylgruppe mit $(C_1-C_{18})$ oder einer $(C_6-C_{18})$ Arylgruppe, und, besagter aromatischer Ring ist möglicherweise C-substituiert durch ein oder mehrere Halogenatome oder eine oder mehrere Gruppen ausgewählt aus $(C_1-C_{18})$ Alkyl; Cyano; Hydroxyl; $(C_1-C_{18})$ Alkoxy; $(C_6-C_{18})$ Aryl-oxy; Carboxy; $(C_1-C_{18})$ Alkoxycarbonyl; Nitro; Trifluoromethyl; $-SO_3M$, wobei M ein Alkalimetall oder ein Wasserstoffatom ist; Amino möglicherweise alkyliert durch eine oder zwei $(C_1-C_{18})$ Alkylgruppen; und $-N^+R_3R_4R_5$ in der $R_3$, $R_4$ und $R_5$ unabhängig voneinander aus $(C_1-C_{18})$ Alkyl ausgewählt werden,
wobei es als vereinbart gilt, dass, wenn die Ar-Gruppe ein quartäres Stickstoffatom umfasst, umfasst es, notwendig für die elektrische Neutralität, außerdem das negative physiologisch akzeptable Gegenion,

genauso wie ihre physiologisch akzeptablen Salze, erhalten durch Einwirken einer Base oder einer Säure mineralischer oder organischer Natur,
**gekennzeichnet dadurch, dass** man in einem geeigneten Lösungsmittel eine Zusammensetzung der Formel II:

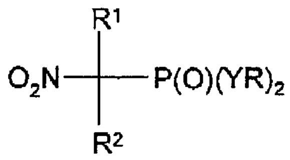

(II)

(in der $R_1$, $R_2$, Y und R wie oben definiert sind), zuvor aktiviert durch eine metallische Zinksuspension in einer wässrigen Ammoniumchloridlösung, mit einem Aldehyd der Formel III reagieren läßt:

Ar-CH=O (III)

(in der Ar wie oben definiert ist).

**10.** Verfahren nach Anspruch 9, **gekennzeichnet dadurch, dass** man die Verbindung der Formel II, die zuvor aktiviert wurde, mit einer Zusammensetzung der Formel III reagieren lässt, bei einer Temperatur zwischen Umgebungstemperatur und 200°C, davon bevorzugt zwischen Umgebungstemperatur und 150°C,

**11.** Verfahren nach irgendeinem der Ansprüche 9 oder 10, **gekennzeichnet dadurch, dass** die vorhergehende Aktivierung der Zusammensetzung der Formel II die Schritte bestehend aus:

(i) Reagieren lassen der Zusammensetzung der Formel II mit metallischem Zink in einem geeigneten Lösungsmittel reagieren lassen in Anwesenheit einer wässrigen Ammoniumchloridlösung, bei einer Temperatur zwischen -10° C und Umgebungstemperatur;

(ii) Filtern der Reaktionslösung, um eine homogene Lösung der Zusammensetzung der aktivierten Formel II zu erhalten, besagte Lösung wird unverändert in dem Verfahren nach irgendeinem der Ansprüche 9 oder 10 verwendet;

umfasst.

**12.** Verfahren nach Anspruch 11, **gekennzeichnet dadurch, dass** das Lösungsmittel im Aktivierungsschritt eine Mischung aus Ethanol/Wasser ist.

**13.** Verfahren für die Herstellung von Zusammensetzungen der allgemeinen Formel (I)

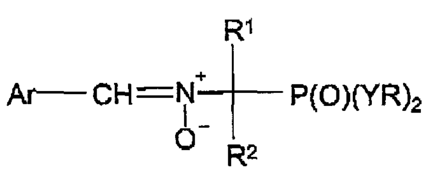

(I)

in der $R_1$ und $R_2$ unabbängig ein ($C_1$-$C_{18}$) Alkyl oder eine Phenylgruppe möglicherweise substituiert durch ($C_1$-$C_{18}$) Alkyl, ($C_1$-$C_{18}$) Alkoxy oder ein Halogenatom, darstellen;

Y stellt O oder $CH_2$ dar;

R stellt ein Wasserstoffatom, eine ($C_1$-$C_{18}$) Alkylgruppe oder eine ($C_6$-$C_{18}$) Arylgruppe dar, und, wenn Y O darstellt, R auch ein Alkalimetall darstellen kann,

Ar stellt einen aromatischen Ring dar, ausgewählt aus einem Phenyl-, Naphthyl-, 2-Pyridyl-, 3-Pyridyl-, 4-Pyridylring oder einem Benzopyridylring, der Formel (i)

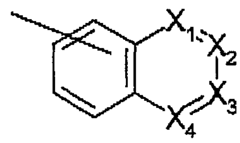

(i)

in der einer von $X_1$, $X_2$, $X_3$ und $X_4$ ein Stickstoffatom darstellt, die anderen ein Kohlenstoffatom darstellen, besagter aromatischer Ring ist möglicherweise C-substituiert durch ein oder mehrere Halogenatome oder eine oder mehrere Gruppen ausgewählt aus ($C_1$-$C_{18}$) Alkyl; Hydroxyl; ($C_1$-$C_{18}$) Alkoxy; Trifluormethyl; ($C_6$-$C_{18}$) Aryloxy; Carboxy; ($C_1$-$C_{18}$) Alkoxycarbonyl; -$SO_3M$, wobei M ein Alkalimetall oder ein Wasserstoffatom ist; Amino möglicherweise alkyliert durch ein oder zwei ($C_1$-$C_{18}$) Alkylgruppen; und -$N^+R_3R_4R_5$ in welcher $R_3$, $R_4$ und $R_5$ unabhängig voneinander aus ($C_1$-$C_{18}$) Alkyl ausgewählt werden,

wobei es als vereinbart gilt, dass, wenn die Ar-Gruppe ein quartäres Stickstoffatom umfasst, umfasst es, notwendig für die elektrische Neutralität, außerdem das negative physiologisch akzeptable Gegenion,
genauso wie ihre physiologisch akzeptablen Salze durch Einwirken einer Base oder einer Säure mineralischer oder organischer Natur erhalten werden,
**gekennzeichnet dadurch, dass** es die Reaktion in einem geeigneten Lösungsmittel von einer Zusammensetzung der Formel II

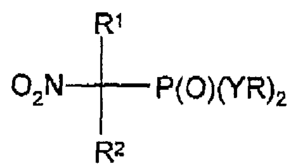

(II)

(in der $R_1$, $R_2$, $R_3$, Y und R wie oben definiert sind)
mit einer Zusammensetzung der Formel III

$$\text{Ar-CH=O} \qquad \text{(III)}$$

(in der Ar wie oben definiert ist) in Anwesenheit metallischen Zinks und Essigsäure, umfasst.

**14.** Verfahren nach Anspruch 13, **dadurch gekennzeichnet, dass** die Reaktion bei einer Temperatur zwischen -10°C und Umgebungstemperatur stattfindet.

**15.** Kosmetische oder pharmazeutische Zusammensetzung umfassend als aktiven Bestandteil wenigstens eine Zusammensetzung nach irgendeinem der Ansprüche 1 bis 8.

**16.** Diagnostische Zusammensetzung, verwendbar für die Auswertung von oxidativem Stress, **gekennzeichnet dadurch, dass** diese wenigstens eine Zusammensetzung nach irgendeinem der Ansprüche 1 bis 8 umfaßt.

**Claims**

**1.** Compounds of general formula I:

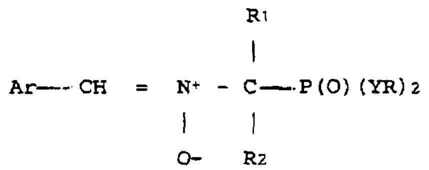

(I)

wherein

$R_1$ and $R_2$ independently represent a ($C_1$-$C_{18}$) alkyl or a phenyl group optionally substituted by ($C_1$-$C_{18}$) alkyl, ($C_1$-$C_{18}$) alkoxy or a halogen atom;
Y represents O or $CH_2$;
R represents a hydrogen atom, a ($C_1$-$C_{18}$) alkyl group or a ($C_6$-$C_{18}$) aryl group and, when Y represents O, R can also represent an alkali metal;
Ar represents an aromatic nucleus chosen from among a phenyl, naphthyl, 2-pyridyl, 3-pyridyl or 4-pyridyl nucleus or a benzopyridyl nucleus of formula (i)

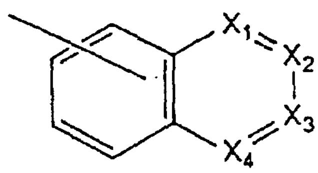

(i)

in which one of the $X_1$, $X_2$, $X_3$ and $X_4$ represents a nitrogen atom, the others representing a carbon atom, the endocylic nitrogen atom of the 2-pyridyl, 3-pyridyl, 4-pyridyl or benzopyridyl nucleus (i) being optionally N-oxidized or substituted by an alkyl group in the $C_1$-$C_{18}$ position or a ($C_6$-$C_{18}$) aryl group and, said aromatic nucleus optionally being C-substituted by one or more halogen atoms or one or more groups chosen from among ($C_1$-$C_{18}$) alkyl; cyano; hydroxyl; ($C_1$-$C_{18}$) alkoxy; ($C_6$-$C_{18}$) aryloxy; carboxy; ($C_1$-$C_{18}$) alkoxycarbonyl; nitro; trifluoromethyl; -$SO_3$M, in which M is an alkali metal or a hydrogen atom; amino optionally alkylated by one or two ($C_1$-$C_{18}$) alkyl groups; and -$N^+R_3R_4R_5$ in which $R_3$, $R_4$ and $R_5$ are chosen independently of one another among ($C_1$-$C_{18}$) alkyl,
it being understood that when the Ar group comprises a quaternary nitrogen atom, it also comprises the physiologically acceptable, negative counterion necessary for electrical neutrality;
as well as their physiologically acceptable salts obtained by the action of a base or a mineral or organic acid.

**2.** Compounds according to claim 1 of formula I in which Ar represents an aromatic nucleus chosen from among phenyl, 2-pyridyl, 3-pyridyl or 4-pyridyl, optionally in N-oxidized form, said aromatic nucleus being optionally C-substituted by a halogen atom, preferably a fluorine atom, or a group chosen from among ($C_1$-$C_{18}$ alkyl; cyano; ($C_1$-$C_{18}$) alkoxy; carboxy; ($C_1$-$C_{18}$) alkoxycarbonyl; nitro; trifluoromethyl; -$SO_3$M, in which M is an alkali metal and preferably sodium and -$N^+(R_3)_3$ in which $R_3$ represents ($C_1$-$C_{18}$) alcohol, it being understood that when the Ar group comprises a quaternary nitrogen atom, it also comprises the physiologically acceptable negative counterion necessary for electrical neutrality, as well as their physiologically acceptable salts obtained by the action of an organic or mineral acid or a base.

**3.** Compounds according to either of the claims 1 and 2, **characterized in that** Ar represents phenyl, optionally substituted in the ortho, meta or para position by a halogen atom or a group chosen from among ($C_1$-$C_{18}$) alkyl, nitro, amino, hydroxyl, ($C_1$-$C_{18}$) alkoxy and ($C_6$-$C_{18}$) aryloxy, as well as their physiologically acceptable salts obtained by the action of an organic or mineral acid or a base.

**4.** Compounds according to either of the claims 1 and 2, **characterized in that** Ar represents an aromatic nucleus chosen from among 2-pyridyl, 3-pyridyl and 4-pyridyl, said aromatic nucleus being optionally substituted on the nitrogen atom by a substituent chosen from among ($C_1$-$C_{18}$) alkyl and ($C_6$-$C_{18}$) aryl, as well as their physiologically acceptable salts obtained by the action of an organic or mineral acid or a base.

**5.** Compounds according to either of the claims 1 and 2, **characterized in that** Ar represents an aromatic nucleus

chosen from among 2-pyridyl, 3-pyridyl and 4-pyridyl, said aromatic nucleus being N-oxidized, as well as their physiologically acceptable salts obtained by the action of a organic or mineral acid or a base.

**6.** Compounds according to any one of the preceding claims, of formula I, in which $R_1$ and $R_2$ independently represent ($C_1$-$C_{18}$) alkyl and preferably ($C_1$-$C_5$) alkyl and Y represents 0, as well as the physiologically acceptable salts obtained by the action of an organic or mineral acid or a base.

**7.** Compounds according to any one of the preceding claims 1, 2 or 4, of formula I, in which when Ar comprises a quaternary nitrogen atom, the counterion is chosen from among a halide, a carbonate, a sulphonate, a sulphate, a phosphate, a phosphonate and a carboxylate such as an oxalate, maleate, citrate, succinate or lactate.

**8.** Compounds according to claim 1, chosen from among:

diethyl-1-(N-benzylidene-N-oxy)amino-1-methyl ethyl phosphonate;
diethyl-1-[N-oxy-N-[4-(N'-oxypyridinyl)formylene]amino]-1-methyl ethyl phosphonate;
diethyl-1-[[N-(4-nitrophenyl)formylene]-N-oxyamino]-1-methyl phosphonate;
diethyl-1-[[N-(4-chlorophenyl)formylene]-N-oxyamino]-1-methyl ethyl phosphonate; and
their physiologically acceptable salts obtained by the action of an organic or mineral acid or a base.

**9.** Process for the preparation of compounds of general formula I

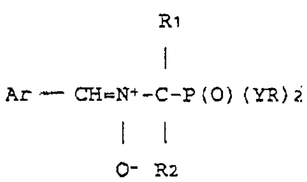

(I)

in which $R_1$ and $R_2$ independently represent a ($C_1$-$C_{18}$) alkyl or a phenyl group, optionally substituted by ($C_1$-$C_{10}$) alkyl, ($C_1$-$C_{18}$) alkoxy or a halogen atom;
Y represents O or $CH_2$;
R represents a hydrogen atom, a ($C_1$-$C_{18}$) alkyl group or a ($C_6$-$C_{18}$) aryl group and, when Y represents O, R can also represent an alkali metal;
Ar represents an aromatic nucleus chosen from among a phenyl, naphthyl, 2-pyridyl, 3-pyridyl, 4-pyridyl nucleus or a benzopyridyl nucleus of formula (i)

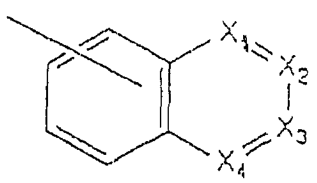

(i)

in which one of the $X_1$, $X_2$, $X_3$ and $X_4$ represents a nitrogen atom, the others representing a carbon atom, the endocyclic nitrogen atom of the 2-pyridyl, 3-pyridyl, 4-pyridyl or benzopyridyl nucleus (i) being optionally N-oxidized or substituted by $C_1$-$C_{18}$ alkyl group or a ($C_6$-$C_{18}$) aryl group, said aromatic nucleus being optionally C-substituted by one or more halogen atoms or one or more groups chosen from among ($C_1$-$C_{18}$) alkyl; cyano; hydroxyl; ($C_1$-$C_{18}$) alkoxy; ($C_6$-$C_{18}$) aryloxy; carboxy; ($C_1$-$C_{18}$) alkoxycarbonyl; nitro; trifluoromethyl, $-SO_3M$, where M is an alkali metal or a hydrogen atom; amino, optionally alkylated by one or two ($C_1$-$C_{18}$) alkyl groups; and $-N^+R_3R_4R_5$, in which $R_3$, $R_4$ and $R_5$ are chosen independently of one another from among ($C_1$-$C_{18}$) alkyl,
it being understood that when the Ar group comprises a quaternary nitrogen atom, it also comprises the physiologically acceptable negative counterion necessary for electrical neutrality;
as well as their physiologically acceptable salts obtained by the action of an organic or mineral acid or a base, **characterized in that** in an appropriate solvent a compound of formula II:

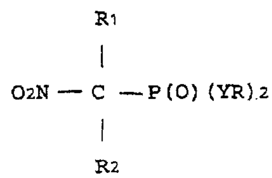 (II)

(in which $R_1$, $R_2$, Y and R are as defined hereinbefore) previously activated by a metallic zinc suspension is reacted in an aqueous ammonium chloride solution with an aldehyde of formula III:

$$Ar\text{-}CH = 0 \qquad (III)$$

(in which Ar is as defined hereinbefore).

**10.** Process according to claim 9, **characterized in that** the previously activated compound of formula II is reacted with a compound of formula III at a temperature between ambient temperature and 200°C and preferably between ambient temperature and 150°C

**11.** Process according to either of the claims 9 and 10, **characterized in that** the prior activation of the compound of formula II comprises stages consisting of:

(i) reacting in an appropriate solvent the compound of formula II with metallic zinc in the presence of an aqueous ammonium chloride solution at a temperature between -10°C and ambient temperature,
(ii) filtering the reaction medium to obtain a homogeneous solution of the compound of formula II in activated form, said solution being used as it is in the process according to either of the claims 9 and 10.

**12.** Process according to claim 11, **characterized in that** the solvent of the activation stage is an ethanol/water mixture.

**13.** Process for the preparation of compounds of general formula I

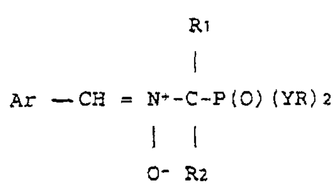 (I)

wherein

$R_1$ and $R_2$ independently represent a ($C_1$-$C_{18}$) alkyl or a phenyl -group, optionally substituted by ($C_1$-$C_{18}$) alkyl, ($C_1$-$C_{18}$) alkoxy or a halogen atom;
Y represents O or $CH_2$;
R represents à hydrogen atom, a ($C_1$-$C_{18}$) alkyl group or a ($C_6$-$C_{18}$) aryl group and, when Y represents O, R can also represent an alkali metal;
Ar represents an aromatic nucleus chosen from among a phenyl, naphthyl, 2-pyridyl, 3-pyridyl, 4-pyridyl nucleus or a benzopyridyl nucleus of formula (i)

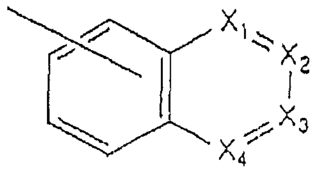

(i)

in which one of the $X_1$, $X_2$, $X_3$ and $X_4$ represents a nitrogen atom, the others representing a carbon atom, said aromatic nucleus being optionally C-substituted by one or more halogen atoms or one or more groups chosen from among $(C_1\text{-}C_{18})$alkyl; hydroxyl; $(C_1\text{-}C_{18})$ alkoxy; trifluoromethyl; $(C_6\text{-}C_{18})$ aryloxy; carboxy; $(C_1\text{-}C_{18})$ alkoxycarbonyl; $-SO_3M$, where M is an alkali metal or a hydrogen atom; amino, optionally alkylated by one or two $(C_1\text{-}C_{18})$ alkyl groups; and $-N^+R_3R_4R_5$, where $R_3$, $R_4$ and $R_5$ are chosen independently of one another from among $(C_1\text{-}C_{18})$ alkyl,
it being understood that when the Ar group comprises a quaternary nitrogen atom, it also comprises the physiologically acceptable negative counterion necessary for electrical neutrality;
as well as their physiologically acceptable salts obtained by the action of an organic or mineral acid or a base, **characterized in that** it comprises the reaction in an appropriate solvent of a compound of formula II

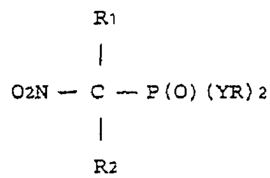

(II)

(in which $R_1$, $R_2$, Y and R are as defined hereinbefore)
with a compound of formula III

$$Ar - CH = O \quad \text{(III)}$$

(in which Ar is as defined hereinbefore)
in the presence of metallic zinc and acetic acid.

**14.** Process according to claim 13, **characterized in that** the reaction takes place at a temperature between -10°C and ambient temperature.

**15.** Cosmetic or pharmaceutical composition comprising as the active ingredient at least one compound according to any one of the claims 1 to 8.

**16.** Diagnostic composition usable in the evaluation of oxidative stress, **characterized in that** it comprises at least one compound according to any one of the claims 1 to 8.